# EUROPEAN PATENT SPECIFICATION

(11) **EP 2 271 393 B1**
(45) Date of publication and mention of the grant of the patent: **17.06.2015**
(21) Application number: 08724150.1
(22) Date of filing: 28.03.2008
(51) Int. Cl.: A61M 25/02, A61N 1/05

(54) **SUTURE SLEEVE**
NAHTHÜLSE
MANCHON DE SUTURE

(43) Date of publication of application: 12.01.2011
(73) Proprietor: St. Jude Medical AB, 175 84 Järfälla (SE)
(72) Inventor: HELGESSON, Marcus, 182 37 Danderyd (SE)
(74) Representative: Sjölander, Henrik
(86) International application number: PCT/SE2008/000230
(87) International publication number: WO 2009/120116

(56) References cited:
- EP-A1- 0 535 285
- WO-A1-2007/024164
- FR-A1- 2 707 175
- US-A- 4 986 815
- US-A- 5 129 405
- US-A- 5 242 431
- US-A- 5 273 053
- US-A- 5 603 730
- US-A- 5 603 730
- US-A- 5 746 722

## Description

The invention relates to a suture sleeve, comprising a tubular body of an elastic material which is adapted to be positioned in a human or animal body and is provided with a through bore to allow introducing of a lead or other elongated and flexible body into the bore for implantation of the lead via the suture sleeve.

### Background of the invention

In the following description and claims, reference is made to use of a suture sleeve, according to the invention, in association with an electrical lead which is implantable into a human or animal body for monitoring and/or controlling of an organ inside the body. However, it is to be understood that a su- ture sleeve according to the invention could be used in association also with other kinds of elongated and flexible bodies, such as e.g. catheters which are implanted for draining purposes or the like.

When implanting an electrical lead into a human or animal body, the distal end of the lead is normally attached to an organ inside the body, whereas the proximal end of the lead is connected to an electrical device for monitoring and/or controlling of the organ, e.g. a pacemaker or a defibrillator for monitoring and/or controlling of the heart function. Normally, it is important that the lead is fixated somewhere along its length to body tissue in order to take up any tensile forces in the lead by the tissue and thereby prevent de- tachment of the lead from the organ. It is also important that the lead-through into for example a blood vessel is performed in a bleeding preventing way in order to prevent inflammatory reactions and the like.

To achieve the above object, it is common practice to use a suture sleeve, which surrounds the lead and which on the one hand is fixated to the lead and on the other hand to the tissue. The fixation to the lead may, in the simplest embodiment, be accomplished by tying a suture wire around the suture sleeve such that it will be deformed and hence clamped around the lead. However, the function of such a clamping suture wire will be very uncertain since it will depend on how hard the physician tightens the suture wire. There is also a risk that the lead can be damaged if the suture wire is tightened to hard.

In prior art it is also known different suture sleeves utilizing various locking mechanisms to achieve fixation of the suture sleeve and the lead in relation to each other. As examples of this kind of suture sleeves can be mentioned e.g. US 5746722 and WO 2007/024164. However, one disadvantage with that type of suture sleeve is that it normally is technically rather complicated and is composed of two or more assembled separate pieces, which will make them rather expensive to manufacture.

In US 5603730 is disclosed a suture sleeve, which is formed as one single unitary piece of an elastic material. The suture sleeve comprises a through bore having a cylindrical central portion, the diameter of which is slightly smaller than the outer diameter of the lead for which it is adapted. To accomplish threading and displacement of the suture sleeve onto the lead, it is provided with a slot in the wall of the central portion, such that when tabs, which projects in opposite directions from the sleeve, is bent relative to each other, the slot will be widened and the inner diameter of the central portion enlarged such that the lead can be displaced inside the suture sleeve. When the tabs are released, the slot will close and the central portion will accordingly be decreased and clamp the lead. One disadvantage with this kind of suture sleeve is that the clamping action around the lead will be rather poor due to the comparatively large slot. Another disadvantage is that the presence of the tabs can cause discomfort for the patient since they may cause abrasions and the like when implanted inside the body.

### Summary of the invention

It is an object of the invention to provide a suture sleeve, which can reliably clamp around a lead or other elongated, flexible body and in a simple way be released from the lead to allow displacement along the lead and which can be manufactured to a low cost. At least this object is achieved by a suture sleeve according to claim 1.

Accordingly, the basis of the invention is the understanding that the above object may be achieved by means of a suture sleeve being manufactured of a resilient material and provided with a through bore which, in at least a portion thereof, is formed with a non-circular cross section such that the inner walls of the through bore, in a non-manipulated state, will abut the outer surfaces of the lead and hence fixate the same. However, in a manipulated state when the suture sleeve is deformed by compressing the suture sleeve in opposite directions by means of fingers or a tool, the suture sleeve will be deformed such that the through bore will be enlarged and thereby allow dis- placing of the lead and the suture sleeve in relation to each other. For example if the cross section of the through bore has an oval shape in a non-manipulated state, it will adopt a more circular cross section in a manipulated state. If, on the other hand, the cross section of the through bore has a rhomboid shape in a non-manipulated state, it will adopt a more square shape in a manipulated state. As soon as the grip around the suture sleeve is relieved, the suture sleeve will strive to resume its original shape such that the inner walls of the through bore will abut the outer surfaces of the lead and hence prevent or at least counteract the mutual displacement of the suture sleeve and the lead in relation to each other.

Naturally, the invention can be modified in many different ways within the overall idea. It is preferred that the suture sleeve is manufactured as one single unitary piece of one single material and in a single manufacturing step, e.g. by injection moulding of silicone, in order to reduce costs. However, it would also be possible to manufacture the suture sleeve of two or more dif- ferent pieces which are assembled together. In hereinafter described and illustrated embodiments of the invention, the suture sleeve is provided with a through bore which is non-circular in cross section only in a central portion of the suture sleeve. Two cross sectional shapes of the through bore is disclosed, namely an oval and a rhomboid shape. However, it is to be understood that also other non-circular cross sectional shapes could be conceivable and also the entire through bore of the suture sleeve could have a non-circular cross section. The portion having a non-circular cross section can also be composed of two or more sub portions with different non-circular shapes.

In the illustrated embodiments of the invention, the suture sleeve is provided with ridges along the opposite locations where the suture sleeve is adapted to be compressed when manipulating it in order to enlarge the cross section of the through bore. The ridges serve at least two main purposes. On the one hand they stiffen the suture sleeve along the length of the non-circular portion such that when compressing the suture sleeve by means of the fingers or a tool, essentially the entire non-circular portion will be deformed such that a lead easily can be displaced through the bore. On the other hand the ridges indicate for the physician where to apply the compressive force in order to open up the through bore. However, it is to be understood that the suture sleeve can be provided with also other types of stiffening and/or indicating means, such as a stiffening means in form of incorporated bars of e.g. metal and indicating means in form of e.g. flat surfaces or colour markings.

In the hereinafter disclosed embodiments, the suture sleeve is provided with two suture wire grooves around its circumference and more precisely one suture wire groove on each side of the non-circular portion of the suture sleeve. These suture wire grooves are primarily adapted to allow fixation of the suture sleeve to the tissue, e.g. a vein and/or muscle tissue, by means of a suture wire without the risk for the suture wire to slip off from the suture sleeve. The fixation of the suture sleeve in relation to the lead is achieved above all by means of the clamping effect of the non-circular bore section. However, if so required, the fixation can be reinforced by tying a suture wire around the suture sleeve in one of the suture wire grooves for deforming the suture sleeve and clamping it around the lead.

In the hereinafter described and illustrated embodiments of the invention, the suture sleeve is used to fixate an electrical lead, which can be used e.g. for monitoring and controlling the function of a heart. However, it is to be understood, as is mentioned before, that the suture sleeve also could be used for fixating and/or sealing of other kinds of implantable elongated and flexible bodies, such as a catheter for drainage or the like.

### Brief description of the drawings

The invention will hereinafter be described with reference to the accompanying drawings, in which:
- Fig 1: is a partly cut through side view illustrating a suture sleeve and a lead being implanted into a body via a vein;
- Fig 2: is a perspective view of a suture sleeve;
- Fig 3: is a longitudinal sectional view through the suture sleeve along the line III-III in fig 5;
- Fig 4: is a longitudinal sectional view through the suture sleeve along the line IV-IV in fig 5;
- Fig 5: is a cross sectional view through the suture sleeve according to fig 2 in a non-manipulated state;
- Fig 6: is a cross sectional view according to fig 3 in a manipulated state;
- Fig 7: is a cross sectional view through a suture sleeve according to an alternative embodiment; and
- Fig 8: is a cross sectional view through a suture sleeve according to a further alternative embodiment adapted for two leads.

### Detailed description of embodiments of the invention

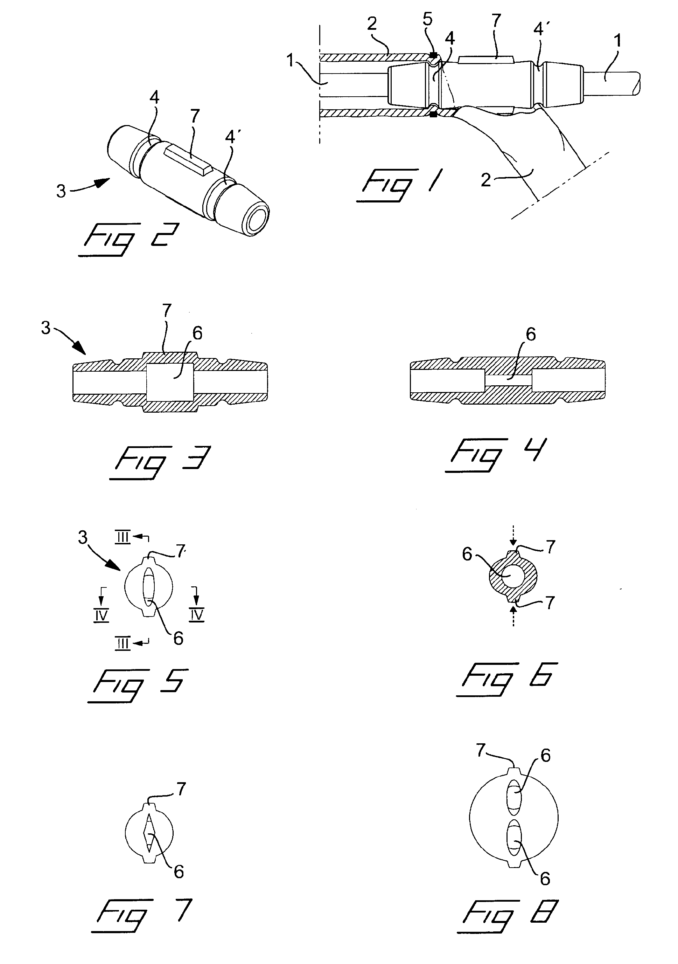

Reference is first made to fig 1, in which is illustrated a situation where a lead 1 is implanted into a not shown body by being introduced into a vein 2, which for this purpose has been cut open. To ensure fixation of the lead in relation to the body, the lead is introduced into a through bore of a suture sleeve 3, one end of which is introduced a short distance into the opening in the vein. It is not necessary that one end of the suture sleeve has to be introduced into the vein. In many cases the lead alone is introduced into the vein, while the suture sleeve is positioned entirely outside the vein and attached to other kind of tissue. The suture sleeve 3, which is illustrated in a perspective view in fig 2, is substantially spool-shaped and is in each of its end portions provided with a circumferential suture groove 4, 4'. A suture wire 5 is tied around the vein 2 and the suture sleeve 3 in the area above the inner suture groove 4. In this way the suture sleeve will be fixated in relation to the vein and hence also to the body.

In order to fixate also the lead 1 in relation to the suture sleeve 3, the suture sleeve, according to the invention, is in a central portion formed with a non-circular through bore 6, as is illustrated in figs 3 to 5. More precisely, the central portion of the through bore is in this case oval shaped in cross section. In this way the central portion of the through bore will squeeze around the lead 1 and prevent displacement of the suture sleeve and the lead in relation to each other. In an alternative embodiment, the transition sections between the central portion, having a non-circular cross section, and each end portion, having a circular cross section, can be made more smoothly than shown in the drawing, e.g. with a conical shape, in order to facilitate introducing of the lead into the through bore.

As can be seen from figs 1, 2 and 5, the suture sleeve is on the outside provided with two opposed stiffening and indicating means in form of ribs 7 along the non-circular through bore 6 in the central portion. More precisely, the stiffening ribs 7 are located along the surface portions of the suture sleeve circumference where the apexes of the oval shaped through bore are located, as apparent from fig 5. When it is desirable to displace the lead and the suture sleeve in relation to each other, it is sufficient to apply pressure in opposite directions on these stiffening ribs 7, by means of the fingers or a tool, such that the suture sleeve will be deformed and the trough bore will be enlarged and adopt a more circular cross section, as is illustrated in fig 6. When the suture sleeve is manipulated in this way, it is easy to perform displacement of the suture sleeve and the lead in relation to each other.

Normally, the clamping effect of the suture sleeve around the lead, as a consequence of the non-circular cross section of the through bore, will provide a sufficient slip resistance between the lead and the suture sleeve for most applications. However, should a need for an increased slip resistance arise, it is possible to tie another suture wire around the suture sleeve in the outer suture groove 4', which will compress the suture sleeve further around the lead.

An alternative embodiment of the invention is illustrated in the cross sectional view of fig 7. Here the central portion of the through bore 6 is formed as a rhomboid in cross section. As with the embodiment according to figs 3-6, the central portion of the through bore will be enlarged and adopt a more square cross section when applying opposite directed pressures on the stiffening ribs 7.

In fig 7 a further alternative embodiment of the invention is illustrated. Here the suture sleeve is somewhat thicker in cross section and comprises two separate through bores in order to enable implantation of two leads via the suture sleeve. Each of the through bores 6 in the central portion of the suture sleeve, has an oval cross sectional form similar to the embodiment of figs 3-6.

## Claims

1. A suture sleeve (3) comprising a tubular body of an elastic material which is adapted to be positioned in a human or animal body and is provided with a through bore (6) to allow introduction of a lead (1) into the through bore (6) for implantation of the lead (1) via the suture sleeve (3), wherein an end of the suture sleeve (3) is adapted to be introduced into an opening in a vein (2) of the human or animal body and wherein said through bore includes two end portions and a central portion (6), whereby each end portion is formed with a circular cross section, **characterized in that** said central portion of the through bore (6) is formed with an oval or rhomboid cross section in a non-manipulated state and **in that** said central portion of the through bore (6) adopts a more circular or square cross section in a manipulated state in which compressing force is applied in opposite directions on the suture sleeve (3).

2. A suture sleeve according to claim 1, **characterized in that** a central portion of the through bore (6) is formed with an oval cross section in a non-manipulated state, whereas each end portion of the through bore (6) is formed with a circular cross section.

3. A suture sleeve according to claim 1 or 2, **characterized in that** it is manufactured in one single unitary piece.

4. A suture sleeve according to any of the preceding claims, **characterized in that** the suture sleeve (3) is provided with ridges (7) at opposed locations wherein application of said compressive force to the ridges results in the suture sleeve adopting said manipulated state.

5. A suture sleeve according to any of the preceding claims, **characterized in that** it is provided with two or more through bores (6) each having a central portion that is, in a non-manipulated state, formed with an oval or rhomboid cross section.

6. A suture sleeve according to any of the preceding claims, **characterized in that** it comprises at least one suture groove (5) around its outer circumference.

## Patentansprüche

1. Nahthülse (3), die einen röhrenförmigen Körper aus einem elastischen Material umfasst, der so ausgelegt ist, dass er in einem Körper eines Menschen oder Tiers platziert wird, und mit einer durchgehenden Bohrung (6) versehen ist, damit das Einführen einer Leitung (1) in die durchgehende Bohrung (6) zum Implantieren der Leitung (1) über die Nahthülse (3) ermöglicht wird, wobei ein Ende der Nahthülse (3) so ausgelegt ist, dass es in eine Öffnung in einer Vene (2) des Körpers des Menschen oder Tiers eingeführt wird, und wobei die durchgehende Bohrung zwei Endabschnitte und einen mittigen Abschnitt (6) aufweist, wobei jeder Endabschnitt mit einem kreisförmigen Querschnitt versehen ist,
**dadurch gekennzeichnet, dass** der mittige Abschnitt der durchgehenden Bohrung (6) in einem nicht betätigten Zustand mit einem ovalen oder rautenförmigen Querschnitt versehen ist und dadurch, dass der mittige Abschnitt der durchgehenden Bohrung (6) in einem betätigten Zustand, in dem in entgegengesetzten Richtungen eine Druckkraft auf die Nahthülse (3) ausgeübt wird, einen eher kreisförmigen oder quadratischen Querschnitt annimmt.

2. Nahthülse nach Anspruch 1, **dadurch gekennzeichnet, dass** ein mittiger Abschnitt der durchgehenden Bohrung (6) in einem nicht betätigten Zustand mit einem ovalen Querschnitt versehen ist, wohingegen jeder Endabschnitt der durchgehenden Bohrung (6) mit einem kreisförmigen Querschnitt versehen ist.

3. Nahthülse nach Anspruch 1 oder 2, **dadurch gekennzeichnet, dass** sie als ein einzelnes einteiliges Teil gefertigt ist.

4. Nahthülse nach einem der vorhergehenden Ansprüche, **dadurch gekennzeichnet, dass** die Nahthülse (3) mit Rippen (7) an gegenüberliegenden Stellen versehen ist, wobei ein Aufbringen der Druckkraft auf die Rippen dazu führt, dass die Nahthülse den betätigten Zustand annimmt.

5. Nahthülse nach einem der vorhergehenden Ansprüche, **dadurch gekennzeichnet, dass** sie mit zwei oder mehr durchgehenden Bohrungen (6) versehen ist, die jeweils einen mittigen Abschnitt aufweisen, der in einem nicht betätigten Zustand mit einem ovalen oder rautenförmigen Querschnitt versehen ist.

6. Nahthülse nach einem der vorhergehenden Ansprüche, **dadurch gekennzeichnet, dass** sie mindestens eine Fadenrille (5) um ihren Außenumfang herum umfasst.

## Revendications

1. Manchon de suture (3) comprenant un corps tubulaire en un matériau élastique qui est adapté pour être positionné dans un corps humain ou animal et qui est doté d'un trou traversant (6) afin de permettre l'introduction d'une dérivation (1) dans le trou traversant (6) pour l'implantation de la dérivation (1) par le biais du manchon de suture (3), une extrémité du manchon de suture (3) étant adaptée pour être introduite dans une ouverture dans une veine (2) du corps humain ou animal et ledit trou traversant comportant deux parties d'extrémité et une partie centrale (6), chaque partie d'extrémité étant formée avec une section transversale circulaire,
**caractérisé en ce que** ladite partie centrale du trou traversant (6) est formée avec une section transversale ovale ou rhomboïdale dans un état non actionné et **en ce que** ladite partie centrale du trou traversant (6) adopte une section transversale plus circulaire ou carrée dans un état actionné dans lequel une force de compression est appliquée sur le manchon de suture (3) dans des directions opposées.

2. Manchon de suture selon la revendication 1, **caractérisé en ce qu'**une partie centrale du trou traversant (6) est formée avec une section transversale ovale dans un état non actionné, tandis que chaque partie d'extrémité du trou traversant (6) est formée avec une section transversale circulaire.

3. Manchon de suture selon la revendication 1 ou 2, **caractérisé en ce qu'**il est fabriqué en une seule pièce unique.

4. Manchon de suture selon l'une quelconque des revendications précédentes, **caractérisé en ce que** le manchon de suture (3) est doté de nervures (7) à des emplacements opposés, l'application de ladite force de compression sur les nervures ayant pour résultat que le manchon de suture adopte ledit état actionné.

5. Manchon de suture selon l'une quelconque des revendications précédentes, **caractérisé en ce qu'**il est doté de deux trous traversants (6) ou plus, chacun possédant une partie centrale qui est, dans un état non actionné, formée avec une section transversale ovale ou rhomboïdale.

6. Manchon de suture selon l'une quelconque des revendications précédentes, **caractérisé en ce qu'**il comprend au moins une rainure de suture (5) autour de sa circonférence extérieure.
